# NOUVEAU FASCICULE DE BREVET EUROPEEN

(11) **EP 0 689 828 B2**
(45) Date de publication et mention de la décision concernant l'opposition: **19.07.2000**
(45) Mention de la délivrance du brevet: 13.11.1996
(21) Numéro de dépôt: 95401060.9
(22) Date de dépôt: 05.05.1995
(51) Int. Cl.: A61K 7/42

(54) **Compositions cosmétiques antisolaires comprenant de la 2,4,6-tris(p-(2'-éthylhexyl-1'-oxycarbonyl)anilino)-1,3,5-triazine et du malate de dioctyle et utilisations**
Kosmetische Sonnenschutzmittel enthaltend 2,4,6-Tris(p-(2'-Ethyl-Hexyl-1'-Oxycarbonyl)Anilino)-1,3,5 Triazine und Dioctyl Malate und Verwendungen
Cosmetic sunscreening compositions containing 2,4,6-tris(p-(2'-ethyl-hexyl-1'-oxycarbonyl)anilino)-1,3,5-triazine and dioctylmalate and uses

(30) Priorité: 03.06.1994 FR 9406834
(43) Date de publication de la demande: 03.01.1996
(73) Titulaire: L'OREAL, 75008 Paris (FR)
(72) Inventeur: Ascione, Jean-Marc, F-75010 Paris (FR); Allard, Delphine, F-92700 Colombes (FR)

(56) Documents cités:
- EP-A- 0 457 687
- Technische Information UVINUL Marken, BASF (août 1992), pp. 8-9
- Chemical Products Desk Reference, M & I Ash. Edward Arnold, London, p. 133

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires à pouvoir photoprotecteur d'une part et propriétés cosmétiques d'autre part améliorés, comprenant, dans un support cosmétiquement acceptable en particulier de type émulsion huile-dans-eau, (i) de la 2,4,6-tris[p-(2'-éthylhexyl-1'-oxycarbonyl)anilino]-1,3,5-triazine à titre de filtre organique solaire lipophile et (ii) une huile particulière convenablement sélectionnée, présente en une quantité déterminée, et qui est du malate de dioctyle.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV). Selon leur caractère lipophile ou au contraire hydrophile, ces filtres peuvent se répartir, respectivement, soit dans la phase grasse, soit dans la phase aqueuse, de la composition finale.

Il s'avère qu'un filtre particulièrement intéressant et largement utilisé à ce jour est constitué par la 2,4,6-tris[p-(2'-éthylhexyl-1'-oxycarbonyl)anilino]-1,3,5-triazine, qui est un filtre lipophile, fortement actif dans l'UV-B, photostable et rémanent à l'eau, et qui est notamment vendu sous la dénomination commerciale de "UVINUL T 150" par la Société BASF. Malgré tout, en l'absence de renfort d'autres filtres, son pouvoir photoprotecteur est assez limité dans les supports cosmétiques habituels contenant des huiles telles que les mono- ou des polyalcools gras oxyéthylénés ou oxypropylénés ("CETIOL HE" de chez Henkel, ou "WITCONOL APM" de chez Witco) et, en outre, les propriétés cosmétiques qui lui sont attachées sont généralement jugées comme insuffisantes.

Le document EP-A-457 687 concerne une émulsion comprenant, dans la phase huileuse, la 2,4,6-tris[p-(2'-éthylhexyl-1'-oxycarbonyl)anilino]-1,3,5-triazine et, dans la phase aqueuse, l'acide benzène 1,4-[(3-méthylidène camphométhyl sulfonique)] ou un de ses sels. Le malate de dioctyle y est mentionné comme composant possible de la phase huileuse, parmi une multitude de composés. Pourtant, le document EP 457 687 ne propose ni suggère d'utiliser le malate de dioctyle en tant que solvant pour le composé de triazine en une quantité suffisante pour solubiliser à lui seul le filtre.

La présente invention vise à résoudre les problèmes ci-dessus.

Ainsi, à la suite d'importantes recherches menées dans le domaine de la photoprotection évoqué ci-dessus, la Demanderesse a maintenant découvert, de façon inattendue et surprenante, qu'il était possible d'améliorer de manière substantielle le pouvoir photoprotecteur d'une part, et les propriétés cosmétiques d'autre part, de la 2,4,6-tris[p-(2'-éthylhexyl-1'-oxycarbonyl)anilino]-1,3,5-triazine contenue dans les compositions cosmétiques antisolaires en associant ce filtre particulier avec une huile spécifique convenablement sélectionnée et qui est du malate de dioctyle, cette huile devant être présente dans lesdites compositions en une quantité suffisante pour solubiliser à elle seule l'ensemble dudit filtre.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) de la 2,4,6-tris[p-(2'-éthylhexyl-1'-oxycarbonyl)anilino]-1,3,5-triazine, à titre de filtre, et (ii) du malate de dioctyle en une quantité suffisante pour solubiliser à lui seul la totalité dudit filtre, des émulsions comprenant de l'acide benzène-1,4-[(3-méthylidène-camphométhylsulfonique)] ou ses sels dans la phase aqueuse étant exclues.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

Un autre objet enfin de la présente invention réside dans l'utilisation du malate de dioctyle pour améliorer le pouvoir photoprotecteur et/ou les propriétés cosmétiques attachés à la 2,4,6-tris[p-(2'-éthylhexyl-1'-oxycarbonyl)anilino]-1,3,5-triazine contenue dans une composition cosmétique antisolaire.

D'autres caractéristiques, aspects et avantages de la présente invention apparaitront à la lecture de la description détaillée qui va suivre.

Comme indiqué précédemment, la 2,4,6-tris[p-(2'-éthylhexyl-1'-oxycarbonyl)anilino]-1,3,5-triazine est un filtre connu en soi, actif dans l'UV-B, se présentant sous une forme solide, et qui est vendu notamment sous la dénomination commerciale de "UVINUL T 150" par la Société BASF. Ce produit répond à la formule (I) suivante: 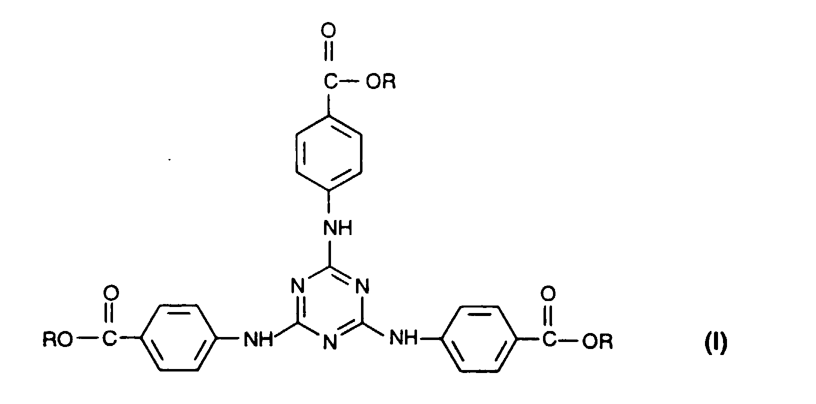 dans laquelle R désigne un radical 2-éthyl hexyle.

Ce filtre peut être présent dans les compositions selon l'invention à une concentration comprise entre 0,1 et 10 % en poids, et de préférence entre 0,5 et 5 % en poids, par rapport au poids total de la composition. Selon une caractéristique importante de la présente invention, ce composé doit se présenter dans la composition antisolaire finale sous une forme totalement, ou substantiellement totalement, solubilisée.

Le malate de dioctyle utilisé dans le cadre de la présente invention est un produit connu en soi, encore appelé Dioctyl Malate dans la nomenclature CTFA (5ième édition, 1993) et qui répond à la formule (II) :

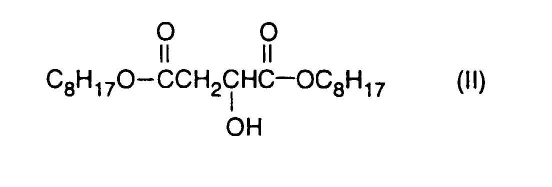

Ce produit est notamment commercialisé sous la dénomination de "CERAPHYL 45" par la Société Mallinckrodt.

Cette huile spécifique utilisée conformément à la présente invention est généralement présente dans les compositions antisolaires finales à des teneurs comprises entre 0,5 et 50 % en poids par rapport au poids total de ladite composition, et de préférence à des teneurs comprises entre 2 et 30 % en poids. Selon une caractéristique essentielle des compositions selon l'invention, le malate de dioctyle doit être utilisé en une quantité telle qu'elle soit suffisante pour solubiliser à elle seule la totalité, ou substantiellement la totalité, de la 2,4,6-tris[p-(2'-éthylhexyl-1'-oxycarbonyl)anilino]-1,3,5-triazine présente dans la composition. Cette quantité minimale en huile solvante destinée à assurer une dissolution complète et stable du filtre solide peut être classiquement déterminée à partir de l'étude des paramètres de solubilité dudit filtre dans ce solvant.

D'une manière générale, on notera que les concentrations en filtre et en huile sont choisies de manière telle que l'indice de protection solaire de la composition finale soit de préférence d'au moins 2.

Enfin, toujours selon un mode préféré de réalisation de la présente invention, le support cosmétiquement acceptable dans lequel se trouvent contenus le filtre et l'huile conformes à l'invention est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que le filtre mentionné ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants et notamment les antioxydants ARL, les opacifiants, les stabilisants, les émollients, les silicones,les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant les filtres ou autres adjuvants hydrophiles) représente généralement de 50 à 95 % en poids, de préférence de 70 à 90 % en poids, par rapport à l'ensemble de la formulation, la phase huileuse (comprenant notamment le ou les filtres et autres adjuvants lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation. Si on le désire, la phase grasse de l'émulsion peut n'être constituée que par l'huile conforme à l'invention, à savoir le malate de dioctyle.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Par rapport aux compositions de l'art antérieur contenant de la 2,4,6-tris[p-(2'-éthylhexyl-1'-oxycarbonyl)anilino]-1,3,5-triazine, les compositions conformes à l'invention, qui contiennent ce même filtre mais associé à du malate de dioctyle en quantité suffisante, présentent un meilleur pouvoir glissant et sont moins collantes à l'application, sont moins grasses tout en étant plus douces.

Des exemples concrets, mais nullement limitatifs, illustrant l'invention, vont maintenant être donnés.

### EXEMPLE 1

On a préparé diverses formulations antisolaires se présentant sous la forme d'une émulsion de type huile-dans-eau et contenant (les quantités sont exprimées en % poids par rapport au poids total de la composition):

| | |
|---|---|
| - 2,4,6-tris[p-(2'-éthylhexyl-1-oxycarbonyl)anilino]-1,3,5-triazine ("UVINUL T150"(filtre solaire) | 10 % |
| - Terpolymère réticulé: acide méthacrylique/acrylate d'éthyle/steareth-10 allyl ether, en émulsion aqueuse à 30 % ("Salcare SC 90" de chez Allied Colloids)(émulsionnant) | 5 % |
| - Huile | 20 % |
| - Conservateurs | qs |
| - Eau qsp | 100% |

en faisant varier la nature de l'huile utilisée.

Chacune de ces émulsions a été réalisée en dissolvant le filtre dans la phase grasse, puis en ajoutant l'émulsionnant dans cette phase grasse portée aux environs de 80°C, et enfin en additionnant sous agitation rapide la phase aqueuse préalablement chauffée à cette même température.

Pour chacune des formulations ainsi préparées, on a ensuite déterminé le facteur de protection solaire (SPF) qui leur était attaché. Celui-ci a été déterminé en utilisant la méthode *in vitro* décrite par B.L DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les compositions des différentes formulations étudiées et les résultats en facteur de protection moyen obtenus sont rassemblés dans le tableau I donné ci-dessous.

A titre comparatif, on a par ailleurs indiqué les résultats obtenus avec une composition exempte d'huile.

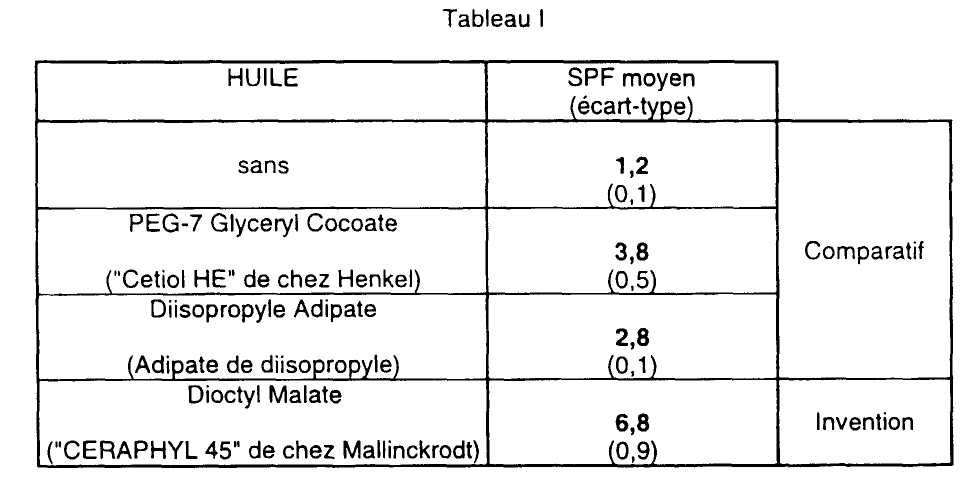

Ces résultats démontrent clairement l'effet bénéfique remarquable apporté par la présence de l'huile conforme à l'invention au niveau des facteurs de protection solaire des compositions finales.

On a par ailleurs comparé les propriétés cosmétiques de la composition conforme à l'invention ci-dessus (UVINUL T 150 + CERAPHYL 45) avec celles de composition comparative UVINUL T 150 + CETIOL HE, toujours telle que préparée ci-dessus. Ces propriétés cosmétiques ont été évaluées par trois personnes-témoins (test d'analyse sensorielle) sur les avant-bras desquelles on a appliqué 0,2 g de composition. Ces personnes ont alors noté de 0 à 5 les effets obtenus sur la peau, la note 5 étant la note maximum correspondant au critère recherché (moins collant, moins gras, plus glissant,....). Les moyennes des notes obtenues (avec leurs écart-types) sont rassemblées dans le tableau II ci-dessous.

**Tableau II**

| **Propriété cosmétique** | **Composition selon l'invention** | **Composition comparative** |
|---|---|---|
| Glissant à l'application | 2,83 (0,76) | 1,17 (0,29) |
| Gras à l'application | 2,83 (0,58) | 2,33 (0,29) |
| Collant à l'application | 2,33 (0,29) | 0,83 (0,76) |
| Frein | 2,83 (0,29) | 1,67 (0,29) |
| Préférence globale | 3,00 (0,5) | 1,67 (0,58) |

Ces résultats démontrent les meilleures propriétés cosmétiques d'usage de la composition selon l'invention.

### EXEMPLE 2

On donne ci-dessous un exemple concret de composition antisolaire conforme à l'invention se présentant sous la forme d'une émulsion huile-dans-eau.

| | |
|---|---|
| - 2,4,6-tris[p-(2'-éthylhexyl-1'-oxycarbonyl)anilino]-1,3,5-triazine ("UVINUL T150") | 3,5 g |
| - 4-tertio-butyl 4'-dibenzoylméthane ("PARSOL 1789" de chez Givaudan)(filtre solaire) | 1 g |
| - TiO₂ de qualité nanopigmentaire ("MT 100 T" de chez Tayca) | 3 g |
| - Dioctyl Malate ("CERAPHYL 45" de chez Mallinckrodt) | 10 g |
| - Mélange d'alcool cétylstéarylique et d'alcool cétylstéarylique oxyéthyléné à 33 moles d'OE (80%/20%) ("DEHSCONET 390" de chez Tensia) | 7 g |
| - Mélange de mono- et distéarate de glycérol ("GELEOL COPEAUX" de chez Gattefosse) | 2 g |
| - Alcool cétylique | 1,5 g |
| - Polydiméthylsiloxane ("SILBIONE HUILE 70 047 V300" de chez Rhône-Poulenc) | 1,5 g |
| - Huile de vaseline | 10 g |
| - Benzoates d'alcools en C12/C15 ("FINSOLV TN" de chez Finetex) | 4 g |
| - Acde éthylène diamine tétraacétique, sel disodique, 2H₂O ("EDETA BD" de chez BASF) | 0,1 g |
| - Glycérine | 20 g |
| - Conservateurs | qs |
| - Parfum | qs |
| - Eau qsp | 100 g |

## Revendications

1. Compositions cosmétiques à usage topique, en particulier pour la photoprotection de la peau et/ou des cheveux, caractérisées par le fait qu'elles comprennent, dans un support cosmétiquement acceptable, (i) de la 2,4,6-tris(p-(2'-éthylhexyl-1'-oxycarbonyl)anilino)-1,3,5-triazine, à titre de filtre, et (ii) du malate de dioctyle en une quantité suffisante pour solubiliser à lui seul la totalité dudit filtre, en excluant des émulsions comprenant de l'acide benzène-1,4-[(3-méthylidène-camphométhylsulfonique)] ou ses sels dans la phase aqueuse.

2. Compositions selon la revendication 1, caractérisées par le fait que ledit filtre est présent à une teneur comprise entre 0,1 et 10 % en poids par rapport au poids total de la composition.

3. Compositions selon la revendication 2, caractérisées par le fait que ladite teneur est comprise entre 0,5 et 5 % en poids.

4. Compositions selon l'une quelconque des revendications précédentes, caractérisées par le fait que le malate de dioctyle est présent à une teneur comprise entre 0,5 et 50 % en poids par rapport au poids total de la composition.

5. Compositions selon la revendication 4, caractérisées par le fait que ladite teneur est comprise entre 2 et 30 % en poids.

6. Compositions selon l'une quelconque des revendications précédentes, caractérisées par le fait que ledit support cosmétiquement acceptable se présente sous la forme d'une émulsion de type huile-dans-eau.

7. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

8. Compositions selon la revendication 7, caractérisées par le fart que lesdits filtres organiques complémentaires sont choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres.

9. Compositions selon l'une quelconque des revendications précédentes, caractérisées par le fait qu'elles comprennent en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

10. Compositions selon la revendication 9, caractérisées par le fait que lesdits pigments ou nanopigments sont choisis parmi les oxydes de titane, de zinc, de fer, de zirconium, de cérium et leurs mélanges, enrobés ou non.

11. Compositions selon l'une quelconque des revendications précédentes, caractérisées par le fait qu'elles comprennent en outre au moins un agent de bronzage et/ou de brunissage artificiel de la peau.

12. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

13. Compositions selon l'une quelconque des revendications précédentes, caractérisées par le fait qu'il s'agit de compositions protectices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

14. Compositions selon l'une quelconque des revendications 1 à 12, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

15. Compositions selon l'une quelconque des revendications 1 à 12, caractérisées par le fait qu'il s'agit de compositions destinées à la protection des cheveux contre les rayons ultraviolets et qu'elles se présentent sous forme de shampooings, de lotions, de gels, d'émulsions, de dispersions vésiculaires non ioniques ou de laques pour cheveux.

16. Compositions selon l'une quelconque des revendications précédentes, caractérisées par le fait qu'elles présentent un indice de protection sur peau d'au moins 2.

17. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

18. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 16.

19. Utilisation du malate de dioctyle pour améliorer le pouvoir photoprotecteur et/ou les propriétés cosmétiques d'une composition cosmétique antisolaire contenant de la 2,4,6-tris[p-(2'-éthylhexyl-1'-oxycarbonyl)anilino]-1,3,5-tria-zine.

## Claims

1. Cosmetic compositions for topical use, in particular for the photoprotection of the skin and/or hair, characterized in that they comprise, in a cosmetically acceptable vehicle, (i) 2,4,6-tris[p-(2'-ethylhexyl-1'-oxycarbonyl)anilino]-1,3,5-triazine, as screening agent, and (ii) dioctyl malate in an amount sufficient to dissolve, on its own, the whole of the said screening agent, excluding emulsions comprising benzene-1,4-[(3-methylidene-camphormethylsulphonic acid)] or its salts in the aqueous phase.

2. Compositions according to Claim 1, characterized in that the said screening agent is present at a content of between 0.1 and 10 % by weight with respect to the total weight of the composition.

3. Compositions according to Claim 2, characterized in that the said content is between 0.5 and 5 % by weight.

4. Compositions according to any one of the preceding claims, characterized in that the dioctyl malate is present at a content of between 0.5 and 50 % by weight with respect to the total weight of the composition.

5. Compositions according to Claim 4, characterized in that the said content is between 2 and 30 % by weight.

6. Compositions according to any one of the preceding claims, characterized in that the said cosmetically acceptable vehicle is provided in the form of an emulsion of oil-in-water type.

7. Compositions according to any one of the preceding claims, characterized in that they additionally comprise one or a number of additional, hydrophilic or lipophilic, organic screening agents which are active in the UV-A and/or UV-B.

8. Compositions according to Claim 7, characterized in that the said additional organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives or polymer screening agents and silicone screening agents.

9. Compositions according to any one of the preceding claims, characterized in that they additionally comprise, as additional photoprotective agents, coated or non-coated metal oxide pigments or nanopigments capable of physically blocking, by scattering and/or reflection, the UV radiation.

10. Compositions according to Claim 9, characterized in that the said pigments or nanopigments are chosen from coated or non-coated titanium oxide, zinc oxide, iron oxide, zirconium oxide, cerium oxide and their mixtures.

11. Compositions according to any one of the preceding claims, characterized in that they additionally comprise at least one agent for the artificial bronzing and/or tanning of the skin.

12. Compositions according to any one of the preceding claims, characterized in that they additionally comprise at least one adjuvant chosen from fatty substances, organic solvents, ionic or non-ionic thickeners, softeners, antioxidants and in particular AFR antioxidants, opacifying agents, stabilizers, emollients, silicones, α-hydroxy acids, anti-foaming agents, moisturizing agents, vitamins, fragrances, preservatives, surface-active agents, fillers, sequestering agents, polymers, propellants, basifying or acidifying agents or dyes.

13. Compositions according to any one of the preceding claims, characterized in that it concerns protective compositions for the human skin or anti-sun compositions and in that they are provided in the form of non-ionic vesicular dispersions or emulsions, in particular emulsions of oil-in-water type, creams, milks, gels, cream gels, suspensions, dispersions, powders, solid sticks, foams or sprays.

14. Compositions according to any one of Claims 1 to 12, characterized in that it concerns make-up compositions for the eyelashes, eyebrows or skin and in that they are provided in the form, anhydrous or aqueous, solid or pasty, of emulsions, suspensions or dispersions.

15. Compositions according to any one of Claims 1 to 12, characterized in that it concerns compositions intended for the protection of the hair against ultraviolet radiation and in that they are provided in the form of shampoos, lotions, gels, emulsions, non-ionic vesicular dispersions or hair lacquers.

16. Compositions according to any one of the preceding claims, characterized in that they have a protection factor on the skin of at least 2.

17. Use of the compositions defined in any one of the preceding claims as, or for the manufacture of, cosmetic compositions for the protection of the skin and/or hair against ultraviolet radiation and in particular solar radiation.

18. Cosmetic treatment process for protecting the skin and/or the hair against ultraviolet radiation, in particular solar radiation, characterized in that it consists in applying an effective amount of a composition as defined in any one of Claims 1 to 16 to the latter.

19. Use of dioctyl malate for improving the photoprotective power and/or cosmetic properties of an anti-sun cosmetic composition containing 2,4,6-tris[p-(2'-ethylhexyl-1'-oxycarbonyl)anilino]-1,3,5-triazine.

## Patentansprüche

1. Kosmetische Zusammensetzungen zur topischen Anwendung, insbesondere zum Lichtschutz der Haut und/oder der Haare,
dadurch gekennzeichnet, daß
sie in einem kosmetisch akzeptablen Träger
(i) 2,4,6-Tris[p-(2'-ethylhexyl-1'-oxycarbonyl)-anilino]-1,3,5-triazin als Lichtschutzfilter und
(ii) Dioctylmalat in zur Solubilisierung der Gesamtmenge des Lichtschutzfilters durch diese Verbindung allein ausreichender Menge
enthalten, wobei Emulsionen ausgenommen sind, die Benzol-1,4-[(3-methylidencamphermethylsulfonsäure)] oder ihre Salze in der wäßrigen Phase enthalten.

2. Zusammensetzungen nach Anspruch 1,
dadurch gekennzeichnet, daß
das Lichtschutzfilter in einem Mengenanteil von 0,1 bis 10 Gew.-%,bezogen auf das Gesamtgewicht der Zusammensetzung, vorliegt.

3. Zusammensetzungen nach Anspruch 2,
dadurch gekennzeichnet, daß
der Gehalt an Lichtschutzfilter 0,5 bis 5 Gew.-% beträgt.

4. Zusammensetzungen nach einem der vorhergehenden Ansprüche,
dadurch gekennzeichnet, daß
das Dioctylmalat in einem Mengenanteil von 0,5 bis 50 Gew.-%, bezogen auf das Gesamtgewicht der Zusammensetzung, vorliegt.

5. Zusammensetzungen nach Anspruch 4,
dadurch gekennzeichnet, daß
der Gehalt an Dioctylmalat 2 bis 30 Gew.-% beträgt.

6. Zusammensetzungen nach einem der vorhergehenden Ansprüche,
dadurch gekennzeichnet, daß
der kosmetisch akzeptable Träger in Form einer Öl-in-Wasser-Emulsion vorliegt.

7. Zusammensetzungen nach einem der vorhergehenden Ansprüche,
dadurch gekennzeichnet, daß
sie ferner ein oder mehrere hydrophile oder lipophile organische Lichtschutzfilter enthalten, die im UV-A-Bereich und/oder im UV-B-Bereich wirksam sind.

8. Zusammensetzungen nach Anspruch 7,
dadurch gekennzeichnet, daß
die ergänzenden organischen Lichtschutzfilter ausgewählt sind unter Zimtsäurederivaten, Salicylsäurederivaten, Campherderivaten, Triazinderivaten, Benzophenonderivaten, Dibenzoylmethanderivaten, β,β-Diphenylacrylat-Derivaten, p-Aminobenzoesäurederivaten, polymeren Lichtschutzfiltern und Silicon-Lichtschutzfiltern.

9. Zusammensetzungen nach einem der vorhergehenden Ansprüche,
dadurch gekennzeichnet, daß
sie ferner als ergänzende Lichtschutzstoffe Pigmente oder Nanopigmente von Metalloxiden enthalten, die gegebenenfalls ummantelt sind und befähigt sind, die UV-Strahlung physikalisch durch Streuung und/oder Reflexion zu blockieren.

10. Zusammensetzungen nach Anspruch 9,
dadurch gekennzeichnet, daß
die Pigmente oder Nanopigmente unter Titanoxid, Zinkoxid, Eisenoxid, Zirkoniumoxid, Ceroxid sowie deren Gemischen ausgewählt sind, wobei sie gegebenenfalls ummantelt sind.

11. Zusammensetzungen nach einem der vorhergehenden Ansprüche,
dadurch gekennzeichnet, daß
sie ferner mindestens ein künstliches Hautbräunungsmittel und/oder Hautbraunfärbungsmittel enthalten.

12. Zusammensetzungen nach einem der vorhergehenden Ansprüche,
dadurch gekennzeichnet, daß
sie ferner mindestens einen Hilfsstoff enthalten, der ausgewählt ist unter ionischen und nichtionischen Verdickungsmitteln, Mitteln für die Geschmeidigkeit, Antioxidantien und insbesondere ARL-Antioxidantien, Trübungsmitteln, Stabilisatoren, Mitteln für die Weichheit, Siliconen, α-Hydroxysäuren, Antischaummitteln, Feuchthaltemitteln, Vitaminen, Parfums, Konservierungsmitteln, grenzflächenaktiven Mitteln, Füllstoffen, Sequestrierungsmitteln, Polymeren, Treibmitteln, Alkalinisierungsmitteln oder Ansäuerungsmitteln und Farbstoffen.

13. Zusammensetzungen nach einem der vorhergehenden Ansprüche,
dadurch gekennzeichnet, daß
es sich um Zusammensetzungen zum Schutz der menschlichen Epidermis oder um Zusammensetzungen zum Lichtschutz handelt und sie in Form von nichtionischen Vesikeldispersionen, Emulsionen, insbesondere Emulsionen vom Öl-in-Wasser-Typ, Cremen, Milchen, Gelen, cremigen Gelen, Suspensionen, Dispersionen, Pudern, festen Sticks, Schäumen oder Sprays vorliegen.

14. Zusammensetzungen nach einem der Ansprüche 1 bis 12,
dadurch gekennzeichnet, daß
es sich um Zusammensetzungen zum Make-up der Wimpern, der Augenbrauen oder der Haut handelt und sie in fester oder pastoser, wasserfreier oder wasserhaltiger Form, in Form von Emulsionen, Suspensionen oder Dispersionen vorliegen.

15. Zusammensetzungen nach einem der Ansprüche 1 bis 12,
dadurch gekennzeichnet, daß
es sich um Zusammensetzungen handelt, die zum Schutz der Haare gegen UV-Strahlung bestimmt sind und die in Form von Shampoos, Lotionen, Gelen, Emulsionen, nichtionischen Vesikeldispersionen oder Haarsprays vorliegen.

16. Zusammensetzungen nach einem der vorhergehenden Ansprüche,
dadurch gekennzeichnet, daß
sie einen Haut-Lichtschutzfaktor von mindestens 2 aufweisen.

17. Verwendung der in einem der vorhergehenden Ansprüche definierten Zusammensetzungen als oder zur Herstellung von kosmetischen Zusammensetzungen zum Schutz der Haut und/oder der Haare gegen UV-Strahlung, insbesondere gegen Sonnenstrahlung.

18. Verfahren zur kosmetischen Behandlung zum Schutz der Haut und/oder der Haare gegen UV-Strahlung, insbesondere gegen Sonnenstrahlung,
dadurch gekennzeichnet, daß
es darin besteht, auf die Haut und/oder die Haare eine wirksame Menge einer Zusammensetzung nach einem der Ansprüche 1 bis 16 aufzutragen.

19. Verwendung von Dioctylmalat zur Verbesserung des Lichtschutzvermögens und/oder der kosmetischen Eigenschaften von kosmetischen Zusammensetzungen zum Lichtschutz, die 2,4,6-Tris [p-(2'-ethylhexyl-1'-oxycarbonyl)-anilino]-1,3,5-triazin enthalten.
